# EUROPEAN PATENT SPECIFICATION

(11) **EP 3 049 792 B1**
(45) Date of publication and mention of the grant of the patent: **25.09.2019**
(21) Application number: 13771432.5
(22) Date of filing: 25.09.2013
(51) Int. Cl.: G01N 21/64, G01N 21/84, G01N 33/00, G01N 33/483

(54) **METHOD AND DEVICE FOR DETERMINING A NUTRITIONAL STATE OF A PLANT**
VERFAHREN UND VORRICHTUNG ZUR BESTIMMUNG EINES ERNÄHRUNGSZUSTANDES EINER PFLANZE
PROCÉDÉ ET DISPOSITIF POUR DÉTERMINER UN ÉTAT NUTRITIONNEL D'UNE PLANTE

(43) Date of publication of application: 03.08.2016
(73) Proprietor: FOSS Analytical A/S, 3400 Hilleroed (DK); University of Copenhagen, 1871 Frederiksberg C (DK)
(72) Inventor: CARSTENSEN, Andreas, DK-1871 Frederiksberg C (DK); FRYDENVANG, Jens, DK-1871 Frederiksberg C (DK); HUSTED, Søren, DK-1871 Frederiksberg C (DK); MAARSCHALKERWEERD, Marie van, DK-4000 Roskilde (DK)
(74) Representative: Hilton, Charles Paul
(86) International application number: PCT/EP2013/069899
(87) International publication number: WO 2015/043623

(56) References cited:
- EP-A1- 2 638 797
- WO-A1-03/081217
- US-A- 6 075 611
- US-A1- 2008 154 512
- HIROSHI OZAKI ET AL: "Quantitative analysis of the relationship between induction kinetics of chlorophyll fluorescence and function of genes in the cyanobacterium Synechocystis sp. PCC 6803", PHOTOSYNTHESIS RESEARCH ; OFFICIAL JOURNAL OF THE INTERNATIONAL SOCIETY OF PHOTOSYNTHESIS RESEARCH, SPRINGER, BERLIN, DE, vol. 101, no. 1, 1 July 2009 (2009-07-01), pages 47-58, XP019733178, ISSN: 1573-5079, DOI: 10.1007/S11120-009-9462-Y
- MERZ D ET AL: "Chlorophyll fluorescence biosensor for the detection of herbicides", FRESENIUS JOURNAL OF ANALYTICAL CHEMISTRY, SPRINGER, BERLIN, DE, vol. 354, 1 January 1996 (1996-01-01), pages 299-305, XP002206792, ISSN: 0937-0633
- CHRISTIAN RITZ ET AL: "Functional Regression Analysis of Fluorescence Curves", BIOMETRICS, vol. 65, no. 2, 21 July 2008 (2008-07-21), pages 609-617, XP055090819, ISSN: 0006-341X, DOI: 10.1111/j.1541-0420.2008.01097.x
- BOISVERT S ET AL: "Quantitative analysis of the experimental O-J-I-P chlorophyll fluorescence induction kinetics", FEBS JOURNAL, vol. 273, no. 20, 20 September 2006 (2006-09-20), pages 4770-4777, XP055368143, GB ISSN: 1742-464X, DOI: 10.1111/j.1742-4658.2006.05475.x
- SCHMIDT S B ET AL: "Latent manganese deficiency in barley can be diagnosed and remediated on the basis of chlorophyll a fluorescence measurements", PLANT AND SOIL, KLUWER ACADEMIC PUBLISHERS, NL, vol. 372, no. 1, 11 May 2013 (2013-05-11), pages 417-429, XP035333416, ISSN: 0032-079X, DOI: 10.1007/S11104-013-1702-4 [retrieved on 2013-05-11]
- HELLAND I S ET AL: "Related versions of the multiplicative scatter correction method for preprocessing spectroscopic data", CHEMOMETRICS AND INTELLIGENT LABORATORY SYSTEMS, ELSEVIER SCIENCE PUBLISHERS B.V. AMSTERDAM, NL, vol. 29, no. 2, 1995, pages 233-241, XP004037865, ISSN: 0169-7439, DOI: 10.1016/0169-7439(95)00031-1
- SCHMITZ P ET AL: "Evaluation of the nodulated status of Vigna unguiculata probed by the JIP-test based on the chlorophyll a fluorescence rise", Science Access 3 (1), 2001, XP055458134, DOI: 10.1071/SA0403684 Retrieved from the Internet: URL:http://www.publish.csiro.au/sa/pdf/SA0 403684 [retrieved on 2018-03-09]
- BALLI T ET AL: "Classification of biological signals using linear and nonlinear features;Classification of biological signals using linear and nonlinear features", PHYSIOLOGICAL MEASUREMENT, INSTITUTE OF PHYSICS PUBLISHING, BRISTOL, GB, vol. 31, no. 7, 26 May 2010 (2010-05-26), pages 903-920, XP020194634, ISSN: 0967-3334, DOI: 10.1088/0967-3334/31/7/003
- PERREAULT F ET AL: "Effect of cadmium on photosystem II activity in: alteration of O-J-I-P fluorescence transients indicating the change of apparent activation energies within photosystem II", PHOTOSYNTHESIS RESEARCH ; OFFICIAL JOURNAL OF THE INTERNATIONAL SOCIETY OF PHOTOSYNTHESIS RESEARCH, SPRINGER, BERLIN, DE, vol. 107, no. 2, 28 December 2010 (2010-12-28), pages 151-157, XP019880445, ISSN: 1573-5079, DOI: 10.1007/S11120-010-9609-X

## Description

The present invention provides a method of determining a nutritional state of plant according to claim 1. The present invention provides an instrument for determining a nutritional state of a plant according to claim 12.

### BACKGROUND OF THE INVENTION

Photosynthesis is a physiological process that is fundamental for the functioning of a plant. A measure of the photosynthesis performance of a plant is therefore a valuable source of information for determining the physiological state of the plant. Photosynthesis converts absorbed light energy into chemical energy that can be used by the plant, and is performed by complex processes in which chlorophyll plays an essential role. While a large part of the light energy absorbed by the plant goes to photosynthesis, some of it undergoes non-photochemical quenching (for a large part through heat dissipation) or is re-emitted by the chlorophyll as fluorescence. Since the three mechanisms - photosynthesis, non-photochemical quenching and chlorophyll fluorescence - are supplied by the same energy source, a variation in performance of one of these processes due to biotic or abiotic factors will be reflected by a variation in at least one of the other processes. However, measuring the complete energy balance for these processes is not practically possible. Nevertheless, the fluorescence signal contains a wealth of information. However, it is a very challenging task to disentangle this information and derive information from fluorescence studies about the physiological state of a plant, which is specific with respect to the influence of a particular parameter, such as deficiency of a particular nutrient or group of nutrients.

The photosynthesis process is subject to a particular kinetics which is reflected in the time dependence of the chlorophyll a fluorescence. Upon excitation with actinic light, the chlorophyll a fluorescence increases on a time-scale of milliseconds up to about a second from a background fluorescence intensity F₀ up to a maximum fluorescence intensity Fₘ, and subsequently rolls off on a time-scale of minutes. This phenomenon is called fluorescence induction and can be observed most clearly for leaves that have been dark-adapted initially so as to allow for the largest possible increase in photosynthesis performance. The rising portion of the fluorescence induction curve exhibits a series of plateaus, commonly denoted by the letters O-J-I-P, and may thus be referred to as the OJIP-rise. Due to the link between chlorophyll a fluorescence transients and the photosynthesis process, analysing these transients, and the OJIP-rise in particular, can be used to provide a benchmark test on the overall performance of a given plant. However, it is generally very difficult to isolate the specific influence of a particular parameter on the fluorescence induction transient of a given plant and derive any specific information on the state of the plant, e.g. with respect to a specific nutrient. Exceptionally, it has proven possible to directly link the ratio of the difference between maximum and minimum intensity (Fᵥ) and the maximum intensity (Fₘ) to the nutritional status of manganese - due to the very direct role manganese plays in the photosynthesis (Husted et al., Plant Physiology, 2009, pp. 825-833). Other approaches suggest a detailed theoretical analysis of the physical mechanisms involved in the photosynthesis processes to retrieve information on the overall health of the plant from an analysis of the fluorescence induction transients (Stirbet et al., Journal of Photochemistry and Photobiology B: Biology, 2011, pp. 236-257). While justified in scientific studies targeted towards understanding the details of the photosynthesis processes, such physical models are not as yet available for implementation in an instrument targeted to determining the nutrient specific nutritional state of a plant. Apart from the above-mentioned method regarding manganese, available methods are generally not suited for distinguishing at least to some level the influences of different nutrients at an early stage of development of the plant, in the field, and with results on the spot. Such information would be desirable, e.g. for facilitating micro-management of fertilization to improve agricultural crops while avoiding excess usage. If available at an early stage, such information would allow for corrective measures before crop damage is permanent.

Therefore, there is a need for a method of determining the nutritional state of a plant with respect to one or more nutrients, which addresses or overcomes at least some of the above-mentioned challenges. The method should at least to some degree be capable of distinguishing between influences of different nutrients. Preferably, the method should be suited for implementation in a mobile measurement instrument suited for field use.

WO 03/081217 A1 discloses a method and apparatus for investigating one or more nutritional deficiencies, e.g. manganese or boron deficiencies, based on the time-resolved measurement of chlorophyll fluorescence and comparison of fluorescence parameters such as maximum fluorescence values with reference values.

In "Evaluation of the nodulated status of Vigna unguiculata probed by the JIP-test based on the chlorophyll a fluorescence rise", Science Access 3(1), 2001, Schmitz et al. present the assessment of nitrate deficiency based on detection of K-peak height in the O-K-J-I-P curve of chlorophyll a fluorescence. Data processing includes double normalization followed by subtraction of a reference curve.

In "Latent manganese deficiency in barley can be diagnosed and remediated on the basis of chlorophyll a fluorescence measurements", Plant Soil 372:417-429 (2013), Schmidt et al. present the characterization of Mn deficiencies of barley plants by analysis of chlorophyll a fluorescence induction kinetics.

### SUMMARY OF THE INVENTION

According to the invention, a method of determining a nutritional state of a plant with respect to one or more nutrients according to claim 1 is provided.The method comprises the steps of:
- recording a time series of a fluorescence induction signal of a tissue sample of the plant using a fluorometer device to obtain a signal data time series, wherein the signal data time series at least comprises signal data within the rising portion of the chlorophyll a fluorescence induction signal, and
- determining the nutritional state from an empirical model applied to the signal data time series, wherein the empirical model is based on pre-recorded reference data time series pre-processed to enhance non-linear components thereof by suppressing lower order components thereof and relates nutritional states to changes in non-linear components of the time-dependent progression of the similarly pre-processed signal data time series.

In the context of the present application, the term 'nutrient' refers to a chemical element that is considered essential for a plant to complete a full life cycle. The term 'nutrient' thus includes the elements oxygen (O), hydrogen (H), carbon (C), nitrogen (N), phosphorus (P), potassium (K), calcium (Ca), sulphur (S), magnesium (Mg), boron (B), chlorine (CI), manganese (Mn), iron (Fe), zinc (Zn), copper (Cu), molybdenum (Mo) and nickel (Ni).

The nutritional state of a plant with respect to one or more nutrients can be described as healthy, when the plant contains sufficient amounts of these nutrients in order to function properly. If the plant only contains insufficient amounts of one or more nutrients, the lack of nutrient affects the health of the plant, and the nutritional state is described as deficient. The method according to the invention monitors the functioning of the photosynthesis process by recording the fluorescence transient and relating variations in the shape of the fluorescence transient curve to variations in nutrient concentrations in the plant. Consequently, the method detects the 'bioactive' part of the nutrient concentration, which is the part that has an effect on the functioning of the plant - in this case the photosynthesis. A plant at any given age is deficient of a nutrient, when the bioactive concentration is insufficient for the plant to function properly. As such, this method is able to detect deficient nutritional states with respect to one or more nutrients.

Time resolved fluorescence induction data may be obtained using a continuous excitation fluorometer such as the "Handy PEA" commercially available from Hansatech Instruments; or any fluorescence induction measuring device providing a time-resolved fluorescence induction curve with a time-resolution sufficient to resolve the shape of the O-J-I-P features in the rising portion of the fluorescence induction transient. In such an instrument, a tissue sample, such as a leaf, is illuminated by actinic light that activates and saturates photosystem II of the tissue sample. The resulting fluorescence is then collected by a detector capable of measuring the fluorescence intensity emitted by the tissue sample as a function of time. In the "Handy PEA" instrument, the actinic light is provided by an LED-source with a spectral maximum at a wavelength of about 650nm and an intensity of 3000 micro-moles photons/(m² * s), and using optical filters to block the LED-source, the chlorophyll a fluorescence is collected at wavelengths above 650nm using a PIN-photodiode as detector. The spectral range of detection should include a significant portion of the spectral range of chlorophyll a fluorescence emission in order to provide a reliable representation of induction kinetics. The chlorophyll a fluorescence has a pronounced maximum at a wavelength of about 680nm, and a typical range of detection would therefore include this maximum, such as up to 700nm, or even up to 800nm.

The onset of actinic light defines the origin of the time axis, and the fluorescence response from the tissue sample is recorded as a function of time with respect to this origin. Typically, the fluorescence is recorded at intervals that increase with time to account for the logarithmic nature of the fluorescence transient curve exhibiting a fast rise and a slow decline. Typical time intervals between subsequent fluorescent measurements range from 10µs (microseconds) in the beginning to about 10ms (milliseconds) at the maximum of the transient, and may further increase in a logarithmic fashion along the slow decline of the transient.

The method relies on pre-recorded reference data. The reference data is a set of fluorescence transients obtained from plants prepared with a variety of reference nutritional states with respect to one or more nutrients. The reference states should reflect the range of nutritional states to be tested for. By design, the reference data thus contains the information on how the different nutritional states of the plant affect the fluorescence induction signal. As mentioned above, the chlorophyll a fluorescence may be affected by numerous biotic and abiotic factors simultaneously and the wealth of information contained by the fluorescence signal tends to conceal any specific information on the influence of a particular nutrient. The present invention solves this by identifying that differences in shape related features in the time-dependent progression of the signal data are associated to different nutritional states, and utilizing an empirical model to interpret these differences. The term 'shape-related' refers to structures in the progression of the time-dependent fluorescence induction signal; 'shape-related features' are such structures that carry relevant information - here in the form of changes in the shape of the fluorescence induction transient that occur as a function of varying nutritional states. The empirical model is based on the pre-recorded reference data obtained for the various reference nutritional states, and is constructed from the reference data using e.g. multivariate analysis techniques. Based on this empirical model, it is possible to predict the nutritional state of a plant with respect to the nutrient from the signal data.

One of the important merits of the present invention is that shape-related features of the fluorescence induction transient, in particular during the fast OJIP-rise, may be analysed to yield information on the nutritional state of a plant with respect to a particular nutrient or group of nutrients. A further merit of the invention is that, by analysing the shape of the fluorescence induction transient, such information on the nutritional state of a plant may even be independent of genotype and plant species. As a consequence, reference data from one genotype or species may be validated as a reference for signal data of plants of a different one. Yet a further merit of the invention is that an analysis of shape-related features in the fluorescence induction transient yields nutrient-specific information about latent nutrient stress of a plant at a very early stage thus allowing a nutrient-specific treatment of the plant. Early detection of a nutrient deficiency followed by an adequate specific treatment enables correction with a minimum of crop damage, and hence yield loss, as compared to other methods. Yet a further merit of the invention is that a nutrient-specific quantitative analysis of the bioactive level may be achieved.

For some nutrients, the empirical model may allow for a direct and unique identification of the nutritional state with respect to one particular nutrient with a high level of reliability. Examples of such nutrients are phosphorus (P), copper (Cu), manganese (Mn), and sulphur (S). For other nutrients, the empirical model may at least provide a prediction that a plant has a nutrient deficiency, wherein the nutrient may be one of a group of nutrients. Examples of such nutrients are nitrogen (N), potassium (K), calcium (Ca), magnesium (Mg), and iron (Fe). Nevertheless, for these other nutrients, the prediction provides information on the nutritional state of the plant, and reduces the ambiguity in identifying the relevant nutrients. The remaining ambiguity may be lifted by providing additional information. Such additional information may be available beforehand, and allow for eliminating some of the other nutrients in that group. Alternatively such additional information may be sought in supplementary analyses.

The method allows for providing a fast, reliable, and accurate test result from a nondestructive/non-invasive optical measurement that may be performed directly on the plant. Preferably, the empirical model is "pre-constructed" and readily available at the time of testing, i.e. when the nutritional state of the plant under test is to be determined. In this case, only the parameters of the empirical model and not the full range of reference data need to be stored for the test. The reference data need only be accessed again when the empirical model needs to be re-validated/reconstructed. This further reduces the need for processing power and data storage capacity, and is therefore particularly advantageous for implementation in mobile devices and for providing a fast test result.

The method further comprises the step of pre-processing the signal data to enhance non-linear components thereof, wherein the empirical model is constructed from correspondingly pre-processed reference data.

Non-linear features of the time dependent progression of the signal data are enhanced by removing a background of lower order components, thereby enhancing modelling of the nutrient dependent differences between samples in these non-linear features using linear multivariate analysis techniques. Prior to analysis, the same pre-processing is applied to the time-dependent fluorescence induction transients of the signal data as the one applied to the time-dependent fluorescence induction transients of the reference data when constructing the empirical model. The method relies for the analysis of the shape-related features on the information contained in the non-linear features. Variations in the bioactive levels of nutrients in the plant, and thus changes in the nutritional state of the plant, are detected as variations in the non-linear components of the corresponding fluorescence induction transients. Thereby, a vastly improved analysis of "shape-related features" is achieved.

Further according to one embodiment of the method, pre-processing may comprise multiplicative scatter correction (MSC), standard normal variate (SNV) transformation, and/or differentiation. Non-linear components in the time-dependent progression of the fluorescence induction signal are enhanced by suppressing lower-order components. For example, this may be achieved by differentiation with respect to time to suppress linear and sub-linear components. The first derivative removes a constant background. The second derivative removes any linear background. Thereby any non-linear components of the time-dependent progression of the fluorescence induction signal are made more prominent, i.e. enhanced. Similarly MSC and SNV tend to suppress differences in lower-order components between time series. As a consequence, non-linear components become more prominent, i.e. they are enhanced.

Further according to one embodiment of the method, determining the nutritional state comprises classifying the sample in a classification scheme with respect to the one or more nutrients on the basis of the empirical model. Thereby, the method is adapted to provide a classification of the plant under test with regard to its nutritional state as an output. Such output is particularly advantageous in micro-managing agriculture, e.g. in order to detect any unhealthy state, identify the particular nutrients or group of nutrients concerned, and decide on corrective actions to be taken based on that output.

The classification scheme may comprise different levels of detail, e.g. depending on the predictive power of the empirical model with regard to the particular nutrients or group of nutrients. A classification may be specific for one particular nutrient, and/or comprise a remaining ambiguity by determining a deficiency for at least one out of a group of nutrients. The classification may be performed according to quantitative or semi-quantitative information, such as a number representative of a reliability of the prediction, or classes of deficiency (e.g. slightly/moderately/heavily deficient), wherein the classification scheme may be calibrated against the pre-recorded reference data, e.g. by multivariate analysis techniques.

Further according to one embodiment of the method, determining the nutritional state comprises providing a quantitative prediction representative of a bioactive concentration of the one or more nutrients in the plant on the basis of the empirical model. Thereby, the method is adapted to provide a quantitative output with regard to the nutritional state of the plant under test. The quantitative prediction is calibrated against the pre-recorded reference data obtained on reference tissue samples with a known/measured bioactive concentration of one or more nutrients in the plant. Calibration may be performed using any adequate technique, such as by multivariate analysis techniques. The quantitative output may be consolidated into a value representing the bioactive level of one or more nutrients. A quantitative output is advantageous in order to provide a detailed diagnosis of the plant with regard to its nutritional state. Furthermore, a quantitative output has the advantage that any measures of correction can be adjusted to avoid oversupply of nutrients, and that the uptake of available nutrients by the plant can be monitored.

Further according to one embodiment of the method, the empirical model is constructed from the reference data using a multivariate analysis technique selected from the group of partial least squares regression (PLS), or principal component analysis (PCA). In one variant, the partial least squares regression may be a partial least squares discriminant analysis (PLS-DA).

Further according to one embodiment of the method, the empirical model is constructed by training an artificial neural network using the reference data as a training set. The artificial neural network may be trained to recognise any patterns present in the reference data, which relate changes in the shape-related features to changes in the nutritional state with respect to a particular nutrient or group of nutrients.

Further according to one embodiment, the method further comprises the step of selecting a sub-set of the recorded reference and signal data from one or more time intervals. The method may be optimized with respect to a particular nutrient or a particular group of nutrients by selecting a sub-set of the reference and signal data from one or more time-intervals. The sub-set is selected with regard to shape-related features in the time-dependent fluorescence induction signal that are expected to carry information on the nutritional state of the plant with respect to a particular nutrient or group of nutrients, or with regard to shape-related features that have been identified beforehand to carry such information, e.g. by studying the shape of fluorescence induction transients in the reference data with regard to variations in the nutritional state for this particular nutrient or group of nutrients. Preferably, the selection is made so as to enhance the influence of the shape-related features for this particular nutrient or group of nutrients on the empirical model. Thereby, the empirical model is configured for determining a nutritional state for a particular nutrient or a particular group of nutrients by selecting a sub-set of the data.

One way of identifying shape-related features in the time series of the fluorescence induction curves is recording a set of reference data on tissue samples that have been cultivated to contain varying bioactive concentrations of one or more nutrients (the nutrients of interest), and identifying changes in shape by comparing traces from tissue samples with different bioactive concentrations. Shape-related changes may be enhanced by pre-processing to improve prominence of the features, e.g. differentiation prior to comparison. Sub-sets are then selected from particular time intervals where these shape-related features are prominent.

Further according to one embodiment of the method, the reference and signal data is selected in the range between 10ms and 1s, alternatively between 15ms and 100ms, or between 20ms and 50ms.

According to one advantageous embodiment, the signal data and the corresponding reference data are selected from the time interval between 0s and 10s, covering the full OJIP rise and the peak at P including the beginning of the slow decline.

According to a further advantageous embodiment, the signal data and the corresponding reference data are selected from the time interval between 0s and 3s, covering the full OJIP rise.

According to one preferred embodiment, the signal data, and the corresponding reference data are selected from a time-range covering the so-called 'I-step' in the OJIP rise of the fluorescence induction transient. The I-step is a shape-related feature that comprises information on the nutritional state with respect to a number of nutrients. For example, a pronounced change in shape of this feature is observed for latent phosphorus deficiency. Suitable time-intervals covering the I-step are for example in the range between 10ms and 1s, alternatively between 15ms and 100ms, or between 20ms and 50ms.

For some nutrients, such as phosphorus (P), copper (Cu) and sulphur (S), a high level of reliability of predictions of the nutritional state is observed in particular around the I-step. The information carried by this shape-related feature thus allows for direct and unique determination of the nutritional state with respect to these nutrients - including a quantitative prediction of the bioactive concentration of these nutrients based on the same data.

Further according to one embodiment of the method, the one or more nutrients are selected from the group of nitrogen (N), phosphorus (P), potassium (K), calcium (Ca), sulphur (S), magnesium (Mg), boron (B), manganese (Mn), iron (Fe), zinc (Zn), and copper (Cu).

Further according to one embodiment of the method, the one or more nutrients are selected from the group of phosphorus (P), copper (Cu), manganese (Mn), and sulphur (S). Surprisingly, a high level of reliability for nutrient-specific prediction is observed for these nutrients, thus entailing a low risk of confusion with other nutrients

There is provided an instrument configured for performing the method according to any of the above mentioned embodiments. Further embodiments of instruments for determining a nutritional state of a plant with respect to one or more nutrients are cited in the following. The same advantages as mentioned above with respect to the method for determining a nutritional state are achieved. In particular, the instrument allows for providing a fast, reliable, and accurate test result from a non-destructive/non-invasive optical measurement that may be performed directly on the plant. Furthermore, the instrument may be a mobile device suited for use in the field.

According to the invention, an instrument for determining a nutritional state of a plant with respect to one or more nutrients according to claim 12 is provided, comprising
- a fluorometer device configured for recording a time series of a chlorophyll a induction signal of a tissue sample of the plant to obtain a signal data time series, wherein the signal data time series at least comprises signal data within the rising portion of the fluorescence induction signal, and
- an analysis device configured for determining the nutritional state of the plant by applying an empirical model to the signal data time series, wherein the empirical model is based on pre-recorded reference data time series pre-processed to enhance non-linear components thereof by suppressing lower order components thereof and which relates nutritional states to changes in non-linear components of the time-dependent progression of the similarly pre-processed signal data time series.

Further according to one embodiment of the instrument, the analysis device generates an output representative of the nutritional state of the plant. The output may be directly displayed to present data on the nutritional state of the plant under test to a user. The output may be used for deriving a health index for the plant, wherein the health index may be nutrient specific or nutrient-group specific. The instrument may provide the output at an interface for use by a subsequent device, such as a device for diagnosing and/or treating the plant under test. For critical threshold levels, i.e. if a critical state of the plant is determined, the instrument may generate an alarm triggered by the output.

Further according to one embodiment of the instrument, the nutrient specific output is a classification according to a classification scheme based on the empirical model.

Further according to one embodiment of the instrument, the output is a quantitative prediction representative of a bioactive concentration of the one or more nutrients in the plant on the basis of the empirical model.

Further according to one embodiment of the instrument, the one or more nutrients are selected from the group of nitrogen (N), phosphorus (P), potassium (K), calcium (Ca), sulphur (S), magnesium (Mg), boron (B), manganese (Mn), iron (Fe), zinc (Zn), and copper (Cu).

Advantageously according to a further embodiment of the instrument, the one or more nutrients are selected from the group of phosphorus (P), copper (Cu), manganese (Mn), and sulphur (S).

### BRIEF DESCRIPTION OF THE DRAWINGS

In the following the invention is further explained by way of examples and with reference to the drawings. The drawings show on
- Fig.1: Time series of the fluorescence induction signal recorded from four tissue samples with different phosphorus nutritional states,
- Fig.2: The data of Fig.1 after pre-processing by section wise differentiation in each of the time windows A-F to enhance shape-related features,
- Fig.3: Median time series of the fluorescence induction signal recorded from a plurality of tissue samples with a specific nutrient deficiency as well as healthy control plants.
- Fig.4: The data of Fig.3 after pre-processing by section wise differentiation in each of the time windows A-E to enhance shape-related features,
- Fig.5: a PLS prediction of phosphorus concentration (ppm) against independently measured phosphorus concentration (ppm),
- Fig.6: a PCA score plot for the first two principal components PC1 and PC2 of a PCA model based on MSC pre-processed data around the I-step.

### DETAILED DESCRIPTION / EXAMPLES

### Recording of fluorescence induction transients

In all examples, chlorophyll a fluorescence transient measurements were performed using the commercially available Hansatech Instrument "Handy PEA". Using the dark adaption clips fitting this instrument, the tissue samples to be analysed were initially dark adapted for at least 20 minutes, thereby effectively stopping all photosynthesis activity and maximizing the intensity increase of the OJIP-rise. When measuring, the sensor unit of the Handy PEA is placed on the dark adaption clip, thereby allowing the tissue sample to be exposed to the sensor and diodes without letting light in. Initially, a background level was determined by using a short flash of non-actinic light and measuring the response from the tissue sample, and the gain of the detector was adjusted accordingly.

After this initial adjustment, the actual measurement was conducted by illuminating the exposed leaf by three red LED's that are optically filtered to a maximum wavelength of 650nm. This light is on for the duration of the measurement, and irradiates the tissue sample with a photon flux of at least 3000 µmol m⁻² s⁻¹ so as to effectively saturate the tissue sample with actinic light during the measurement.

Switching on the actinic light induces a chlorophyll a fluorescence induction signal with a fast rise of the fluorescence intensity, followed by a slow decline. The fluorescence signal occurs at wavelengths above the wavelength of the actinic light. The intensity of the fluorescence transient was recorded using a PIN photodiode as a detector. Optical filtering is used to ensure that only the longer wavelength (>650nm) fluorescence signal is recorded, thereby avoiding artefacts stemming from actinic light reaching the detector. The PIN-detector integrates the intensity of the induced fluorescence signal over the full spectral range reaching the detector.

A time series of fluorescence intensity measurements was recorded at increasing intervals between successive measurements as a function of total time evolved, wherein the origin of the total time axis is defined as the point when actinic light is switched on. The following gives an overview of the number of data points recorded for a set of signal data, and intervals between successive data points in different time windows of the total time evolved after switching on the actinic light. The chosen distribution of time intervals is one example of a somewhat logarithmic increase of the time intervals with total time evolved. However, other distributions may easily be conceived by the skilled person in order to adapt the time resolution of the recorded signal data with respect to the overall logarithmic nature of the progression of the fluorescence intensity as a function of total time evolved.

| Data point number | Interval / s | Total time / s | Time window |
|---|---|---|---|
| 1-30 | 10 x 10⁻⁶ | (0,0-0,30) x 10⁻³ | A |
| 31-57 | 0,10 x 10⁻³ | (0,40-3,0) x 10⁻³ | B |
| 58-84 | 1,0 x 10⁻³ | (4,0-30) x 10⁻³ | C |
| 85-111 | 10 x 10⁻³ | 0,04-0,30 | D |
| 112-138 | 0,10 | 0,40-3,0 | E |
| 139-145 | 1,0 | 4,0-10 | F |

For the purpose of the present examples each fluorescence induction data set thus consists of 145 individual measurements, and provides a time-dependent fluorescence curve that is best visualized using a logarithmic time-scale. The data sets cover the first 10 seconds of the fluorescence induction transient including the fast fluorescence rise, the peak intensity, and the beginning of the slow decline. When merely studying the fluorescence rise up to and including the intensity peak, only the first approximately 3 seconds are recorded, of which the first second may suffice.

### Reference nutritional states

Two experiments where performed providing two sets of reference nutritional states, each covering a range of physiological states of spring barley (cv. Quench) with different levels of phosphorus deficiency. In both cases, barley plants were grown hydroponically, thereby allowing for a clear control of the nutrient-levels available to each plant. Plants were divided into four different treatments that varied slightly between the two experiments, but in both cases they consisted of one control treatment (P0), and three P-treatments (P1-P3) with decreasing P concentration.

In both experiments, spring barley, cv. Quench, was germinated for eight days in soaked Sorbix vermiculite in a greenhouse with minimum day/night temperatures at 18/15 °C and 16 hours of light each day (minimum 250-300 µmol photons m⁻² s⁻¹), and subsequently grown hydroponically in 4 L opaque cultivation units. The nutrient solution in the growing units is based on a standard control treatment with: 200 µM KH₂PO₄, 200 µM K₂SO₄, 300 µM MgSO₄>7H₂O, 100 µM NaCl, 300 µM Mg(NO₃)₂> 6H₂O, 900 µM Ca(NO₃)₂>4H₂O, 600 µM KNO₃, 50 µM Fe(III)-EDTA-Na, 0.8 µM Na₂MoO₄>2H₂O, 1 µM MnCl₂>4H₂O, 0.7 µM ZnCl₂, 0.8 µM CuSO₄>5H₂O, 2 µM H₃BO₃. To avoid EDTA poisoning, the concentrations of the micronutrients were however halved in the first growth week after transfer to the cultivation units.

Each cultivation unit was continuously aerated with filtered air and the nutrient solution was renewed weekly to ensure optimal nutrient availability of all essential nutrients, except phosphorus for the plants where phosphorus deficiency is induced. pH was kept constant at 6.0±0.3 using ultrapure HCl.

The Control treatment had ample amounts of all nutrients during the whole experiment, and P1, P2, and P3 treatments were supplied with decreasing amounts of P. In both experiments, the intention of the P1 level was to estimate the P level, so that P1 just fulfilled the P requirement of the plants, while avoiding the luxury uptake found in Control plants. Based on previous experience, the concentration of phosphorus in the P1 units was set at 89 µM (i.e. 89 µM of KH₂PO₄). The P2 levels were set at 50% of the P1 level in both experiments, and the P3-level was set at 10 % and 25% of the P1 level in experiment 1 and 2 respectively. Specifically for experiment 2, the potassium removed when reducing the concentration of KH₂PO₄ was replaced by adding additional KCI - thereby keeping a constant level of potassium throughout the experiment across all four treatments.

### Experiment 1 - Climate chamber

The germinated plants were transferred to 32 cultivation units, each containing 10 plants, and divided into two groups (A and B) with 16 units each (day 1). Group A was cultivated in a climate chamber under normal light settings (400 µmol photons m⁻² s⁻¹) and a constant temperature of 20 °C during the whole experiment. Group B was cultivated in a climate chamber with the same initial settings as A, however, when the reduced P levels were induced, the settings were changed into high light intensity (750 µmol photons m⁻² s⁻¹) and a constant temperature at 15 °C. Twice a week the positions of the units were randomized within each chamber.

The 16 units in each climate chamber were divided into the four different P treatments (Control, P1, P2, and P3). For the first 10 days, P1, P2, and P3 units were all supplied with nutrient solution P1, to avoid a luxury uptake of P in the pre-cultivation phase but allow the production of healthy biomass. After 10 days, the three limited P levels described above were induced. At day 21, all three P-limited treatments were replaced by a nutrient solution containing no phosphorus.

The plants were sampled twice in each climate chamber during the experimental period. The first sampling was conducted at day 21 and the last sampling at day 28 where the P1-3 plants had been completely deprived of phosphorus for seven days. At each sampling, the youngest fully developed leaf from five different plants (which were subsequently harvested) in each cultivation unit were analysed using the Handy PEA, and leaves from each separate cultivation unit were subsequently pooled together as one sample and analysed using Inductively Coupled Plasma Optical Emission Spectroscopy (ICP-OES). This way, the average phosphorus-content of the youngest fully mature leaf from each cultivation unit is obtained, and this was used as a reference value for each of the five chlorophyll a fluorescence measurements on individual plants in that cultivation unit.

### Experiment 2 - Greenhouse

The germinated plants were transferred to 16 cultivation units each containing 4 plants (day 1), and were cultivated under the same greenhouse conditions as for the germination period. The 16 cultivation units were divided into the four different phosphorus treatments (Control, P1, P2, and P3), and their positions were randomized twice a week.

All four different treatments were given control-level nutrient supply during the first ten days. On day 10, the P1, P2, and P3 levels were induced using their respective nutrient solutions. On day 21, phosphorus was removed completely from the P1-3 treatments. On day 28, all cultivation units were supplied with control-level nutrient concentrations to observe a potential effect of phosphorus re-supply.

The plants were sampled four times during the experimental period, at day 21, day 23, day 28, and day 30. One plant from each cultivation unit was harvested, and chlorophyll a measurements were performed on both the youngest fully developed leaf and the second youngest fully developed leaf. Unlike experiment 1, each leaf was subsequently analysed separately using ICP-OES, thereby giving a specific reference value for each chlorophyll a fluorescence measurement.

### Data-processing

One of the central merits of the present invention rests in the insight that an appropriate analysis of shape-related features of the progression of the fluorescence induction transient, and in particular of the rising portion, yields nutrient-specific information about the state of deficiency with respect to a specific nutrient. To that end, changes in the shape-related features as compared to the unstressed state with respect to the specific nutrient are detected and analysed. According to the present invention, the nutritional state is determined from these changes by constructing an empirical model, which on the basis of reference data relates specific reference nutritional states to shape-related features in the fluorescence induction transients and applying this empirical model to the signal data obtained from a plant under test. The reference data is pre-recorded fluorescence induction time series obtained from reference plants, i.e. plants that are prepared to be in a particular nutritional state with respect to the specific nutrient as described above. The signal data are fluorescence induction time series obtained from a plant under test. The empirical model is constructed using multivariate analysis techniques.

### Pre-processing

Pre-processing was applied to the fluorescence induction data in order to enhance shape-related features. For a given analysis, both signal data and the corresponding reference data used to construct the empirical model were pre-processed using the same pre-processing technique. A number of different pre-processing techniques may be employed. Two of these pre-processing techniques are presented here. In one analysis the same algorithm as commonly used for multiplicative scatter correction of infrared spectra was applied to a sub-set of time-dependent fluorescence induction data selected from a time-range around the I-step (between 2.6ms and 100ms). The multiplicative scatter correction algorithm turned out to work surprisingly well as pre-processing for the purpose of enhancing shape-related features in the progression of the fluorescence induction data. In a further analysis, numerical differentiation was applied by taking the difference in fluorescence signal of two subsequent data points. The differentiation is done section wise for each of the above-mentioned time windows A-F.

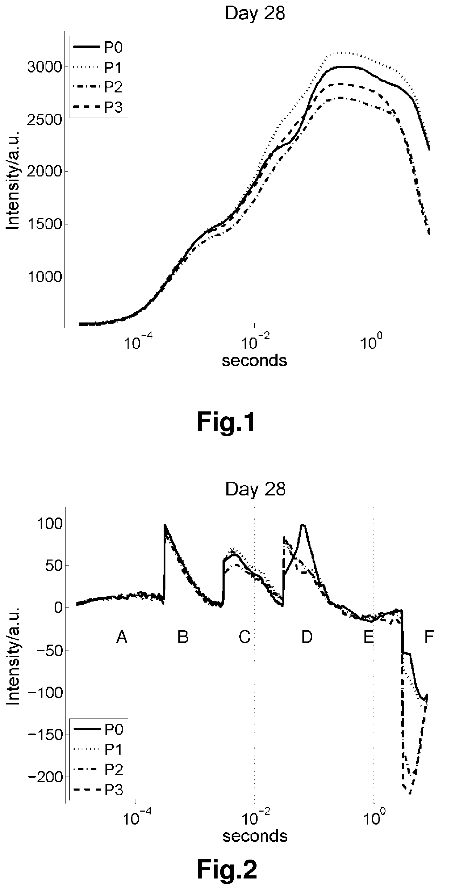

Fig.1 and Fig.2 illustrate the enhancement of the shape-related features by differentiation for different bioactive concentrations of a particular nutrient, here phosphorus. Fig.1 shows the recorded fluorescence induction signal as a function of time for four tissue samples with different phosphorus concentration on a log/linear scale. The data covers the OJIP rise and the beginning of the subsequent decline in the time interval between 0s-10s. Fig.2 shows the corresponding data after pre-processing by section wise differentiation applied to the fluorescence induction signal in each of the time windows A-F. Note for example, the pronounced change in the shape-related feature around the I-step at about 50ms for phosphorus deficient plants (treatments P1-P3) as compared to the healthy state (treatment P0).

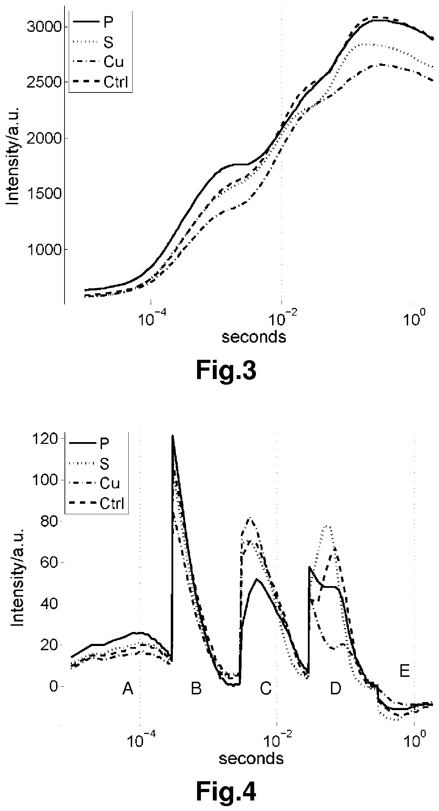

Fig.3 and Fig.4 illustrate the enhancement of the shape-related features by differentiation for different nutrient species. Fig.3 shows the fluorescence induction signal as a function of time from tissue samples with a nutrient specific deficiency on a log/linear scale. The data covers the OJIP rise up to and including the peak at 'P' in the time interval between 0s-3s. Three of the fluorescence induction traces shown are from a plurality of tissue samples in nutrient deficient states with respect to phosphorus, sulphur, and copper, respectively. The fourth fluorescence induction trace is from healthy tissue samples. Fig.4 shows the corresponding data after pre-processing by section wise differentiation applied to the fluorescence induction signal in each of the time windows A-E. Clear nutrient-specific differences in shape are observed for different tissue samples.

### Empirical model

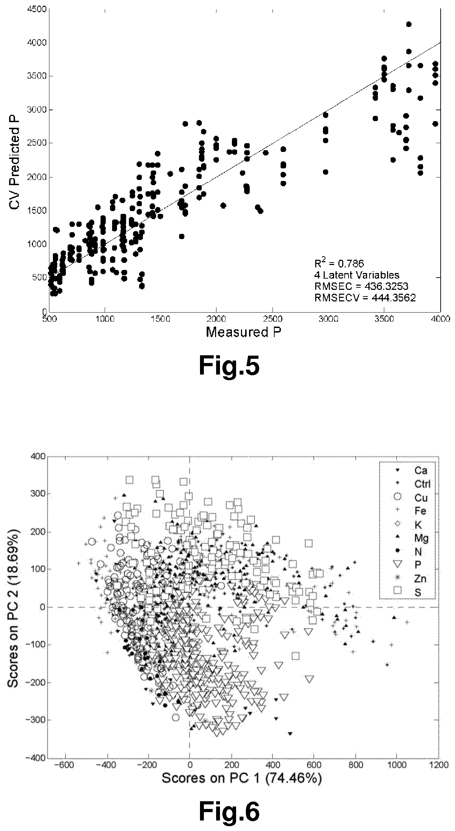

In one analysis, an empirical model is constructed from fluorescence induction transients recorded in the time-range between 0s-10s for the set of reference nutritional states of Experiment 1 and Experiment 2, pre-processed by differentiation. The differentiation is obtained section wise as described above. The empirical model is constructed by Partial Least Squares Regression. A phosphorus concentration of about 3000ppm-4000ppm is the threshold commonly used in practical agriculture for when a plant is considered 'healthy'. A higher phosphorus concentration is thus not 'bioactive' in the plant, and this 'luxury uptake' will instead be stored as a reserve for potential later needs. The empirical model is therefore constructed for all those plants that have an independently measured phosphorus concentration of below 4000ppm. Fig.5 shows a graph of the cross-validated predicted phosphorus concentration (ppm) against measured phosphorus concentration (ppm) for Experiment 1 and Experiment 2. The diagonal line indicates the desired 1:1 relation of perfect prediction. Cross-validation was performed using four randomly selected subsets, and the number of latent variables was chosen to minimize the difference between the root mean squared error of calibration (RMSEC) and the RMSE of cross-validation (RMSECV). Outliers were removed based on Hotelling's T² vs. residual plots. The reliability of the prediction is characterized by an R²-value of about 0.8 indicating a good correlation between measured and predicted phosphorus concentration. Furthermore, the phosphorus concentrations above 3000 ppm (measured) are seen to be slightly under-predicted. This 'cut-off' of the phosphorus concentration predicted on the basis of fluorescence induction data as compared to the total phosphorus concentration in the plant as measured by an independent method, is in agreement with the fact that the fluorescence induction is sensitive to the bioactive fraction of the nutrients contained in the plant rather than the total concentration. As the threshold for barley is between 3000-4000 ppm, it is in full agreement with theory that samples should tend to be underestimated when the phosphorus concentration is above 3000ppm.

In a further analysis, an empirical model is constructed from a collection of fluorescence induction transients recorded for the set of reference nutritional states of Experiment 1 and Experiment 2 with respect to phosphorus and from a library of fluorescence induction transients recorded for deficient nutritional states with respect to nutrients from the group of phosphorus (P), manganese (Mn), boron (B), nitrogen (N), potassium (K), calcium (Ca), sulphur (S), magnesium (Mg), iron (Fe), zinc (Zn), and copper (Cu). A sub-set of the data in the collection is selected by only taking into account fluorescence induction signals around the I-step, in the time interval between 2.6ms and 100ms. The empirical model is constructed using principal component analysis (PCA). Prior to performing the PCA and constructing the empirical model, the fluorescence induction transients were pre-processed by applying the Multiplicative Scatter Correction (MSC) algorithm, which is commonly used for pre-processing of infrared spectra. Such an algorithm is commercially available, for example in the PLS_toolbox 7.3.1 software by Eigenvector Research for Matlab The algorithm acts surprisingly well to enhance shape-related features of the fluorescence induction transients by suppressing lower order artefacts in the time-dependent fluorescence induction signal. PCA is an unsupervised multivariate analysis method. Any pattern detected in the data can therefore give a reliable indication of the presence of systematic dependencies in the data. Fig.6 shows a PCA score plot for the first two principal components PC1 and PC2, wherein PC1 explains 74.5% of the variance in the above mentioned ensemble of data, and PC2 explains 18.7% in this data. Together, PC1 and PC2 thus explain more than 90% of the variance in the data. The plot shows a large number of individual fluorescence induction transients in the ensemble, each represented by a point with the PC1 and PC2 coefficients as the x- and y-coordinates, respectively. Each point thus represents a particular nutritional state with respect to a particular nutrient. Different symbols represent fluorescence induction transients for different nutrients. A clear clustering of phosphorus deficient states is observed in the lower right portion of the graph (hollow triangles). Furthermore, Sulphur deficient states cluster in a region at the top of the graph (hollow squares), and Copper deficient states appear to agglomerate to the left (hollow circles). This plot underlines the capability of the method according to the invention, at least for some nutrients, to uniquely identify a state of nutritional deficiency, and possibly even quantify the level of deficiency.

In yet a further analysis, a partial least squares discriminant analysis (PLSDA) regression is made on the same library of fluorescence induction transients as used for the PCA above. Unlike above however, the entire of fluorescence induction transients in the time interval from 0s-3s is pre-processed by section-wise differentiation. As PLSDA is a supervised method unlike PCA, the results are cross validated by dividing the data into ten random subsets. After removing outliers a PLSDA model is made using 9 latent variables; giving the confusion matrix shown in Table 2, and the confusion table shown in Table 3. These results further corroborate the ability of this method to provide nutrient specific predictions based on shape-related features in the fluorescence induction transients.

**Table 2**

| *Class:* | *TP* | *FP* | *TN* | *FN* |
|---|---|---|---|---|
| Ctrl | 0.53 | 0.14 | 0.86 | 0.47 |
| Ca | 0.27 | 0.03 | 0.97 | 0.73 |
| **Cu** | **0.82** | **0.04** | **0.96** | **0.18** |
| Fe | 0.15 | 0.04 | 0.96 | 0.85 |
| K | 0.19 | 0.01 | 0.99 | 0.81 |
| Mg | 0.13 | 0.01 | 0.99 | 0.87 |
| **Mn** | **0.76** | **0.01** | **0.98** | **0.24** |
| N | 0.40 | 0.04 | 0.96 | 0.60 |
| P | **0.74** | **0.04** | **0.96** | **0.26** |
| Zn | 0.29 | 0.04 | 0.96 | 0.71 |
| **S** | **0.75** | **0.10** | **0.90** | **0.25** |
| B | 0.69 | 0.04 | 0.96 | 0.31 |

Table 2 shows the cross-validated confusion matrix for the PLSDA model (9 latent variables) showing the relative number of true positive (TP), false positive (FP), true negative (TN) and false negative (FN) for each class/nutrient. The nutrients that show promise in terms of specificity are highlighted as bold.

**Table 3**

| *Predicted vs. Actual* | *Ctrl* | *Ca* | *Cu* | *Fe* | *K* | *Mg* | *Mn* | *N* | *P* | *Zn* | *S* | *B* |
|---|---|---|---|---|---|---|---|---|---|---|---|---|
| Predicted as Ctrl | 204 | 0 | 7 | 56 | 8 | 54 | 3 | 0 | 25 | 9 | 17 | 0 |
| Predicted as Ca | 2 | 13 | 6 | 5 | 3 | 14 | 1 | 10 | 7 | 1 | 1 | 0 |
| **Predicted as Cu** | **26** | **7** | **129** | **10** | **4** | **2** | **4** | **1** | **4** | **2** | **1** | **0** |
| Predicted as Fe | 17 | 1 | 7 | 29 | 2 | 9 | 0 | 3 | 10 | 2 | 6 | 0 |
| Predicted as K | 5 | 2 | 0 | 1 | 9 | 5 | 0 | 0 | 2 | 1 | 1 | 2 |
| Predicted as Mg | 9 | 0 | 0 | 4 | 0 | 28 | 0 | 0 | 4 | 0 | 3 | 0 |
| **Predicted as Mn** | **7** | **3** | **0** | **4** | **0** | **3** | **55** | **0** | **6** | **0** | **0** | **0** |
| Predicted as N | 0 | 9 | 0 | 28 | 2 | 22 | 0 | 19 | 3 | 2 | 1 | 1 |
| **Predicted as P** | **9** | **8** | **1** | **9** | **0** | **13** | **5** | **8** | **213** | **4** | **2** | **0** |
| Predicted as Zn | 29 | 4 | 1 | 1 | 13 | 8 | 0 | 4 | 7 | 14 | 0 | 2 |
| **Predicted as S** | **58** | **0** | **7** | **39** | **0** | **48** | **2** | **0** | **1** | **0** | **95** | 0 |
| Predicted as B | 16 | 1 | 0 | 8 | 7 | 3 | 6 | 2 | 6 | 13 | 0 | 11 |

Table 3: Confusion table showing the cross validated most probable predicted classes versus the reference classes. The nutrients that show promise in terms of specificity are highlighted. The PLSDA further supports the capability of the method according to the invention to provide nutrient specific information on the nutritional state of a plant, which at least for some nutrients may even be a unique identification of one or more nutrients related to a deficiency state with a high degree of reliability.

## Claims

1. Method of determining a nutritional state of a plant with respect to one or more nutrients, the method comprising the steps of
- recording a time series of a chlorophyll a fluorescence induction signal of a tissue sample of the plant using a fluorometer device to obtain a signal data time series, wherein the signal data time series at least comprises signal data within the rising portion of the chlorophyll a fluorescence induction signal, and
- determining the nutritional state from an empirical model applied to the signal data time series, **characterised in that** the empirical model is based on pre-recorded reference data time series pre-processed to enhance non-linear components thereof by suppressing lower order components thereof and relates nutritional states to changes in non-linear components of the time-dependent progression of the similarly pre-processed signal data time series.

2. Method according to claim 1, wherein pre-processing comprises one of differentiation, multiplicative scatter correction and standard normal variate transformation.

3. Method according to any of the preceding claims, wherein determining the nutritional state comprises classifying the sample in a classification scheme with respect to the one or more nutrients on the basis of the empirical model.

4. Method according to any of the preceding claims, wherein determining the nutritional state comprises providing a quantitative prediction representative of a bioactive concentration of the one or more nutrients in the plant on the basis of the empirical model.

5. Method according to claim 3 or claim 4, wherein the empirical model is constructed from the reference data time series using a multivariate analysis technique selected from the group of partial least squares regression (PLS), or principal component analysis (PCA).

6. Method according to any of the preceding claims, further comprising the step of selecting a sub-set of the recorded reference data time series and signal data time series from one or more time intervals.

7. Method according to claim 6, wherein the reference data time series and signal data time series is selected in the range between 10ms and 1s after onset of actinic light.

8. Method according to claim 6 wherein the reference data time series and signal data time series is selected in the range between 15ms and 100ms after onset of actinic light.

9. Method according to claim 6 wherein the reference data time series and signal data time series is selected in the range between 20ms and 50ms after onset of actinic light.

10. Method according to any of the preceding claims, wherein the one or more nutrients are selected from the group of nitrogen (N), phosphorus (P), potassium (K), calcium (Ca), sulphur (S), magnesium (Mg), boron (B), manganese (Mn), iron (Fe), zinc (Zn), copper (Cu).

11. Method according to claim 10, wherein the one or more nutrient is selected from the group of phosphorus (P), copper (Cu), manganese (Mn) and sulphur (S).

12. Instrument for determining a nutritional state of a plant with respect to one or more nutrients, the instrument comprising
- a fluorometer device configured for recording a time series of a chlorophyll a fluorescence induction signal of a tissue sample of the plant to obtain a signal data time series, wherein the signal data time series at least comprises signal data within the rising portion of the chlorophyll a fluorescence induction signal, and
- an analysis device configured for determining the nutritional state of the plant by applying an empirical model to the signal data time series, **characterised in that** the empirical model is based on pre-recorded reference data time series pre-processed to enhance non-linear components thereof by suppressing lower order components thereof and which relates nutritional states to changes in non-linear components of the time-dependent progression of the similarly pre-processed signal data time series.

13. Instrument according to claim 12, wherein the analysis device generates an output representative of the nutritional state of the plant.

14. Instrument according to claim 13, wherein the output is a classification according to a classification scheme, wherein the classification is based on the empirical model.

15. Instrument according to claim 13, wherein the output is a quantitative prediction representative of a bioactive concentration of the one or more nutrients in the plant on the basis of the empirical model.

16. Instrument according to any one of claims 13-15, wherein the one or more nutrients are selected from the group of nitrogen (N), phosphorus (P), potassium (K), calcium (Ca), sulphur (S), magnesium (Mg), baron (B), manganese (Mn), iron (Fe), zinc (Zn), and copper (Cu).

## Patentansprüche

1. Verfahren zum Bestimmen eines Ernährungszustands einer Pflanze bezüglich eines oder mehrerer Nährstoffe, wobei das Verfahren die folgenden Schritte umfasst
- Aufzeichnen einer Zeitreihe eines Chlorophyll *a-*Fluoreszenzinduktionssignals einer Gewebeprobe der Pflanze unter Verwendung einer Fluorometervorrichtung, um eine Signaldaten-Zeitreihe zu erhalten, wobei die Signaldaten-Zeitreihe mindestens Signaldaten innerhalb des ansteigenden Abschnitts des Chlorophyll *a*-Fluoreszenzinduktionssignals umfasst, und
- Bestimmen des Ernährungszustands aus einem empirischen Modell, das auf die Signaldaten-Zeitreihe angewendet wird, **dadurch gekennzeichnet, dass** das empirische Modell auf zuvor aufgezeichneten Bezugsdaten-Zeitreihen beruht, die vorverarbeitet wurden, um nichtlineare Komponenten derselben durch Unterdrücken von Komponenten niedrigerer Ordnung derselben zu verstärken, und Ernährungszustände Änderungen nichtlinearer Komponenten der zeitabhängigen Progression der ähnlich vorverarbeiteten Signaldaten-Zeitreihen zuordnet.

2. Verfahren nach Anspruch 1, wobei das Vorverarbeiten eine aus Differentiation, multiplikativer Streukorrektur und Standard Normal Variate Transformation umfasst.

3. Verfahren nach einem der vorhergehenden Ansprüche, wobei das Bestimmen des Ernährungszustands das Klassifizieren der Probe in einen Klassifizierungssystems bezüglich des einen oder der mehreren Nährstoffe aufgrund des empirischen Modells umfasst.

4. Verfahren nach einem der vorhergehenden Ansprüche, wobei das Bestimmen des Ernährungszustands das Bereitstellen einer quantitativen Vorhersage, die für eine bioaktive Konzentration des einen oder der mehreren Nährstoffe in der Pflanze repräsentativ ist, aufgrund des empirischen Modells umfasst.

5. Verfahren nach Anspruch 3 oder Anspruch 4, wobei das empirische Modell aus der Bezugsdaten-Zeitreihe aufgebaut ist, wobei eine Methode der multivarianten Analyse verwendet wird, die aus der Gruppe aus der partiellen Regression der kleinsten Quadrate (PLS) oder der Hauptkomponentenanalyse (PCA) ausgewählt ist.

6. Verfahren nach einem der vorhergehenden Ansprüche, das ferner den Schritt des Wählens einer Untergruppe der aufgezeichneten Bezugsdaten-Zeitreihe und Signaldaten-Zeitreihe aus einem oder mehreren Zeitintervallen umfasst.

7. Verfahren nach Anspruch 6, wobei die Bezugsdaten-Zeitreihe und die Signaldaten-Zeitreihe im Bereich zwischen 10 ms und 1 s nach Beginn des aktinischen Lichts ausgewählt sind.

8. Verfahren nach Anspruch 6, wobei die Bezugsdaten-Zeitreihe und die Signaldaten-Zeitreihe im Bereich zwischen 15 ms und 100 ms nach Beginn des aktinischen Lichts ausgewählt sind.

9. Verfahren nach Anspruch 6, wobei die Bezugsdaten-Zeitreihe und die Signaldaten-Zeitreihe im Bereich zwischen 20 ms und 50 ms nach Beginn des aktinischen Lichts ausgewählt sind.

10. Verfahren nach einem der vorhergehenden Ansprüche, wobei der eine oder die mehreren Nährstoffe aus der Gruppe aus Stickstoff (N), Phosphor (P), Kalium (K), Calcium (Ca), Schwefel (S), Magnesium (Mg), Bor (B), Mangan (Mn), Eisen (Fe), Zink (Zn), Kupfer (Cu) ausgewählt sind.

11. Verfahren nach Anspruch 10, wobei der eine oder die mehreren Nährstoffe aus der Gruppe aus Phosphor (P), Kupfer (Cu), Mangan (Mn) und Schwefel (S) ausgewählt sind.

12. Instrument zum Bestimmen eines Ernährungszustands einer Pflanze bezüglich eines oder mehrerer Nährstoffe, das Instrument umfassend
- ein Fluorometervorrichtung, die zum Aufzeichnen einer Zeitreihe eines Chlorophyll *a*-Fluoreszenzinduktionssignals einer Gewebeprobe der Pflanze konfiguriert ist, um eine Signaldaten-Zeitreihe zu erhalten, wobei die Signaldaten-Zeitreihe mindestens Signaldaten innerhalb des ansteigenden Abschnitts des Chlorophyll *a*-Fluoreszenzinduktionssignals umfasst, und
- eine Analysevorrichtung, die zum Bestimmen des Ernährungszustands der Pflanze konfiguriert ist, indem einen empirisches Modell auf die Signaldaten-Zeitreihe angewendet wird, **dadurch gekennzeichnet, dass** das empirische Modell auf zuvor aufgezeichneten Bezugsdaten-Zeitreihen beruht, die vorverarbeitet wurden, um nichtlineare Komponenten derselben durch Unterdrücken von Komponenten niedrigerer Ordnung derselben zu verstärken, und das Ernährungszustände Änderungen nichtlinearer Komponenten der zeitabhängigen Progression der ähnlich vorverarbeiteten Signaldaten-Zeitreihen zuordnet.

13. Instrument nach Anspruch 12, wobei die Analysevorrichtung eine Ausgabe erzeugt, die für den Ernährungszustand der Pflanze repräsentativ ist.

14. Instrument nach Anspruch 13, wobei die Ausgabe eine Klassifizierung gemäß einem Klassifizierungssystem ist, wobei die Klassifizierung auf dem empirischen Modell beruht.

15. Instrument nach Anspruch 13, wobei die Ausgabe eine quantitative Vorhersage, die für eine bioaktive Konzentration des einen oder der mehreren Nährstoffe in der Pflanze repräsentativ ist, aufgrund des empirischen Modells ist.

16. Instrument nach einem der Ansprüche 13-15, wobei der eine oder die mehreren Nährstoffe aus der Gruppe aus Stickstoff (N), Phosphor (P), Kalium (K), Calcium (Ca), Schwefel (S), Magnesium (Mg), Bor (B), Mangan (Mn), Eisen (Fe), Zink (Zn) und Kupfer (Cu) ausgewählt ist.

## Revendications

1. Procédé pour déterminer un état nutritionnel d'une plante en ce qui concerne un ou plusieurs nutriments, le procédé comprenant les étapes :
- enregistrer une série chronologique d'un signal d'induction de fluorescence de chlorophylle a d'un échantillon de tissu de la plante à l'aide d'un dispositif fluorimètre pour obtenir une série chronologique de données de signal, la série chronologique de données de signal comprenant au moins des données de signal dans la partie montante du signal d'induction de fluorescence de chlorophylle a, et
- déterminer l'état nutritionnel à partir d'un modèle empirique appliqué à la série chronologique de données de signal, **caractérisé par le fait que** le modèle empirique est basé sur une série chronologique de données de référence préenregistrée, prétraitée pour améliorer des composantes non-linéaires de celle-ci par suppression de composantes d'ordre inférieur de celle-ci, et associe des états nutritionnels à des changements de composantes non-linéaires de la progression temporelle de la série chronologique de données de signal prétraitée de manière similaire.

2. Procédé selon la revendication 1, dans lequel le prétraitement comprend l'une parmi une différenciation, une correction de dispersion multiplicative et une transformation de variable aléatoire normale standard.

3. Procédé selon l'une quelconque des revendications précédentes, dans lequel déterminer l'état nutritionnel comprend classer l'échantillon dans un schéma de classification en ce qui concerne le ou les nutriments sur la base du modèle empirique.

4. Procédé selon l'une quelconque des revendications précédentes, dans lequel déterminer l'état nutritionnel comprend fournir une prédiction quantitative représentant une concentration bioactive du ou des nutriments dans la plante sur la base du modèle empirique.

5. Procédé selon la revendication 3 ou la revendication 4, dans lequel le modèle empirique est construit à partir de la série chronologique de données de référence à l'aide d'une technique d'analyse de variables aléatoires multiples choisie parmi le groupe constitué d'une régression des moindres carrés partiels (PLS) ou d'une analyse de composante principale (PCA).

6. Procédé selon l'une quelconque des revendications précédentes, comprenant en outre l'étape de sélection d'un sous-ensemble des série chronologique de données de référence et série chronologique de données de signal enregistrées à partir d'un ou plusieurs intervalles de temps.

7. Procédé selon la revendication 6, dans lequel la série chronologique de données de référence et la série chronologique de données de signal sont choisies dans la plage entre 10 ms et 1 s après l'apparition de lumière actinique.

8. Procédé selon la revendication 6, dans lequel la série chronologique de données de référence et la série chronologique de données de signal sont choisies dans la plage entre 15 ms et 100 ms après l'apparition de lumière actinique.

9. Procédé selon la revendication 6, dans lequel la série chronologique de données de référence et la série chronologique de données de signal sont choisies dans la plage entre 20 ms et 50 ms après l'apparition de lumière actinique.

10. Procédé selon l'une quelconque des revendications précédentes, dans lequel le ou les nutriments sont choisis parmi le groupe constitué de l'azote (N), du phosphore (P), du potassium (K), du calcium (Ca), du soufre (S), du magnésium (Mg), du bore (B), du manganèse (Mn), du fer (Fe), du zinc (Zn), du cuivre (Cu).

11. Procédé selon la revendication 10, dans lequel le ou les nutriments sont choisis parmi le groupe constitué de phosphore (P), de cuivre (Cu), de manganèse (Mn) et de soufre (S).

12. Instrument pour déterminer un état nutritionnel d'une plante en ce qui concerne un ou plusieurs nutriments, l'instrument comprenant :
- un dispositif fluorimètre configuré pour enregistrer une série chronologique d'un signal d'induction de fluorescence de chlorophylle a d'un échantillon de tissu de la plante pour obtenir une série chronologique de données de signal, la série chronologique de données de signal comprenant au moins des données de signal dans la partie montante du signal d'induction de fluorescence de chlorophylle a, et
- un dispositif d'analyse configuré pour déterminer l'état nutritionnel de la plante par application d'un modèle empirique à la série chronologique de données de signal, **caractérisé par le fait que** le modèle empirique est basé sur une série chronologique de données de référence préenregistrée, prétraitée pour améliorer des composantes non-linéaires de celle-ci par suppression de composantes d'ordre inférieur de celle-ci, et qui associe des états nutritionnels à des changements de composantes non-linéaires de la progression temporelle de la série chronologique de données de signal prétraitée de manière similaire.

13. Instrument selon la revendication 12, dans lequel le dispositif d'analyse génère une sortie représentant l'état nutritionnel de la plante.

14. Instrument selon la revendication 13, dans lequel la sortie est une classification selon un schéma de classification, la classification étant basée sur le modèle empirique.

15. Instrument selon la revendication 13, dans lequel la sortie est une prédiction quantitative représentant une concentration bioactive du ou des nutriments dans la plante sur la base du modèle empirique.

16. Instrument selon l'une quelconque des revendications 13 à 15, dans lequel le ou les nutriments sont choisis parmi le groupe constitué de l'azote (N), du phosphore (P), du potassium (K), du calcium (Ca), du soufre (S), du magnésium (Mg), du bore (B), du manganèse (Mn), du fer (Fe), du zinc (Zn) et du cuivre (Cu).
